# EUROPEAN PATENT SPECIFICATION

(11) **EP 4 482 305 B1**
(45) Date of publication and mention of the grant of the patent: **05.11.2025**
(21) Application number: 23705081.0
(22) Date of filing: 08.02.2023
(51) Int. Cl.: A01K 67/36, A01K 67/362

(54) **DEVICE FOR REARING INSECT LARVAE**
VORRICHTUNG ZUR AUFZUCHT VON INSEKTENLARVEN
DISPOSITIF D'ÉLEVAGE DE LARVES D'INSECTES

(30) Priority: 25.02.2022 NL 2031084
(43) Date of publication of application: 01.01.2025
(73) Proprietor: Bob Holtermans Beheer B.V., 5804 VK Venray (NL)
(72) Inventor: HOLTERMANS, Bob Lodewijk Maria, 5804 VK Venray (NL)
(74) Representative: Jilderda, Anne Ayolt
(86) International application number: PCT/IB2023/051116
(87) International publication number: WO 2023/161750

(56) References cited:
- CN-U- 213 523 358
- US-A1- 2019 166 812

## Description

The present invention relates to a device for rearing insects, particularly insect larvae, more particularly fly larvae, still more particularly larvae of the black soldier fly, comprising a rack with at least one elongate rack bottom which extends in the rack between opposite end surfaces thereof and is provided over at least substantially a whole length with a set of upward directed wings on either side.

Insect larvae are increasingly seen as a valuable source of protein in a daily human or animal diet. This makes rearing of insects, more particularly insect larvae, an emerging sector of agriculture, wherein larvae are generally reared from an egg stage to a fully-fledged protein source in a more or less closed rearing space of a hall or a warehouse. Owing to their rapid development and procreation, combined with the capacity to accept a wide range of nutrients, insects are eminently suitable candidates for contributing to solving worldwide nutritional challenges. This applies to insects in general and to flies in particular, wherein the black soldier fly plays a major role.

Chinese utility model CN 213.523.358 discloses a breeding system in which creatures are bred in a multi-layered system of elongated racks, each having an elongate rack bottom which extends between opposite end surfaces thereof and being provided over at least substantially a whole length with a set of upward directed wings on either side.

Insect larvae are usually kept in containers. These are usually low trays in which the insects are raised and are fed. The individual handling of such trays however forms an obstacle in scaling up to a more large-scale production environment. Compared to plant cultivation, the mobility of insect larvae poses an additional challenge here. The insect larvae must be prevented from being able to leave their housing and grow freely into adult insects. It must particularly be prevented that adult insects are able to escape from the rearing space and, being an alien species, in this way endanger the biodiversity in the natural ecosystem. This is why it must be ensured in the usual rearing trays that the larvae are unable to escape therefrom.

The present invention has for its object, among others, to provide a device for rearing insects and/or insect larvae, particularly flying insects such as flies and more particularly black soldier flies, which is both practical and safe in the aforementioned respect.

In order to achieve the stated object a rearing device of the type described in the preamble has the feature according to the invention that the rack bottom is covered with a plastic lining, particularly a polyolefin lining, which also extends laterally over an upward directed part of each of the wings, that the lining is movable over the rack bottom and is removable from the rack at at least one of the end surfaces. The insect larvae can thus be kept on the lining of the rack bottom so that the underlying rack bottom remains protected therefrom. The lining here also provides a base on which and with which the larvae can be processed and transported further. Surprisingly, it has been found that the upright edges of the lining against the upward directed wings of the rack bottom form an insurmountable barrier for the larvae. An additional cover has been found to be unnecessary, whereby the larvae remain readily accessible for inspection, feeding and other treatment during their development. The larvae are nevertheless effectively confined and cannot escape. The invention thus provides a device which is both practical and safe.

With a view to an efficient utilization of a surface area available in the rearing space a preferred embodiment of the rearing device has the feature according to the invention that the rack comprises a number of similar rack bottoms at successive levels one above the other. It has been found that a low height of the collar on the lining already forms a sufficient obstacle for the larvae. Rearing will therefore advantageously take place at multiple levels one above the other, for which purpose this device in each case provides at each level a rack bottom provided with a lining.

With a view to populating the device with fresh insect larvae, a further preferred embodiment of the device has the feature according to the invention that an infeed device is coupled operatively to an inlet of the rack, which infeed device is intended and configured to carry the lining axially into the rack over a rack bottom, and more particularly that the infeed device comprises a dispensing device for supplying at least one of a food source and insect larvae onto an exposed surface of the lining. The infeed device here introduces a fresh lining, optionally together with the insect larvae and/or nutrition dispensed thereon, fully automatically.

It is conversely also desirable that harvesting of the fully grown insect larvae can take place without considerable human intervention as far as possible. For this purpose a further preferred embodiment of the device has the feature according to the invention that an outfeed device is coupled operatively to an outlet of the rack, which outfeed device is intended and configured to carry the lining axially out of the rack, and more particularly that the outfeed device comprises a collecting device for removing the insect larvae from the lining. The invention thus provides the option of a fully integrated system in which both the infeed and outfeed and the rearing cycle of insect larvae are fully automated.

In a particular embodiment the device is characterized according to the invention in that the rack bottoms are substantially formed by a number of substantially parallel bars which extend in axial direction and maintain a mutual intermediate space. The individual bars have been found to provide sufficient support to the lining, while the intermediate spaces which were left open provide for a reduction in the lateral friction in axial direction. The lining will thereby experience less sliding resistance when it is introduced axially over the rack bottom or removed axially thereover.

Although use can in principle be made of a form-retaining lining, for instance in the form of a gutter, a further preferred embodiment of the device has the feature according to the invention that the lining comprises a continuous plastic film, particularly a polyolefin film. Such a cloth can be handled in particularly practical manner, for instance from or onto a roll, and can be arranged on the rack bottom and against the upward directed wings in relatively simple manner. A width of the cloth has here enough excess relative to a corresponding width of the rack bottom that a sufficient collar height can be ensured thereby.

With a view to an adequate support of the lining in the transition from the flat rack bottom to the upward directed wings a further particular embodiment of the device has the feature according to the invention that the wings each comprise a continuous plate body, which plate body comprises an upright wall and a lying bottom, wherein the bottom of each of the wings is connected fixedly to the rack bottom. Besides the desired support, the wings here moreover form a continuous axial guide when the lining is axially introduced or removed.

Although the device can be assembled from various materials, a further preferred embodiment thereof has the feature according to the invention that at least the rack bottom is formed at least substantially from a metal from a group comprising aluminium, stainless steel and optionally preserved steel. Making use of metal for at least the rack bottom(s) ensures a sufficient durability, wear-resistance and thereby lifespan of the device.

The invention also relates to a method for rearing insect larvae, wherein use is made of at least one rearing device according to the present invention, and will be further elucidated hereinbelow with reference to an exemplary embodiment and an accompanying drawing. In the drawing:
Figure 1 is an isometric view of a rack with rack bottoms of an exemplary embodiment of the rearing device according to the invention;
Figure 2 is a top view of a rack bottom together with a schematic representation of an infeed device and outfeed device for the rearing device of figure 1;
Figure 3 is a cross-section of the device of figure 1, provided with a lining according to the invention; and
Figure 4 is an enlarged detail of the device of figure 3.

It is otherwise noted here that the figures are purely schematic and not always drawn to (the same) scale. Some dimensions in particular may be exaggerated to greater or lesser extent for the sake of clarity. Corresponding parts are designated in the figures with the same reference numeral.

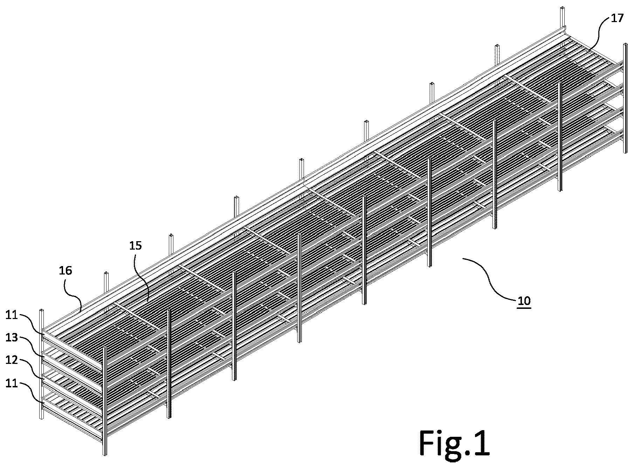

An exemplary embodiment of a rearing device for rearing insect larvae will be further elucidated below with reference to figures 1-4. The device comprises a rack 10 as shown isometrically in figure 1. The rack comprises four levels 11-14 one above the other, with a rack bottom 15 at each level. The whole is composed of aluminium, stainless steel or steel profiles and interconnected by means of welding, bolts and/or rivets to form the assembly shown in the figure.

On a first end side the rack bottoms 15 are provided with an end plate 17, while the opposite side provides an inlet/outlet 18 for respectively product infeed and product outfeed. The rack bottoms 15 extend axially in the rack over substantially a whole length thereof. The rack bottoms 15 are here flanked on both sides by upward directed wings 16 connected thereto. In contrast to rack bottoms 15 themselves, use is for the wings made of a continuous plate body with a flat part which is connected to rack bottom 15 and an upward directed wall which provides a lateral boundary of the rack bottom. Rack bottoms 15 comprise therebetween a system of parallel profiles which maintain a mutual intermediate space.

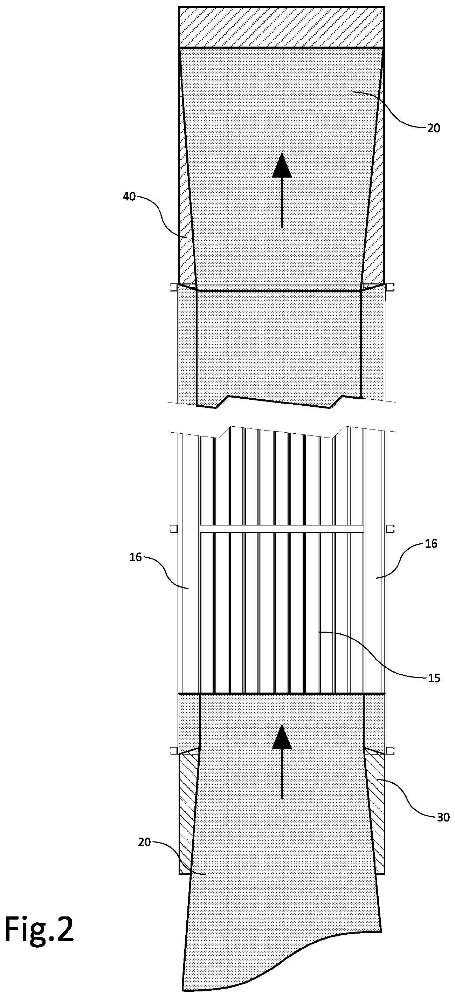

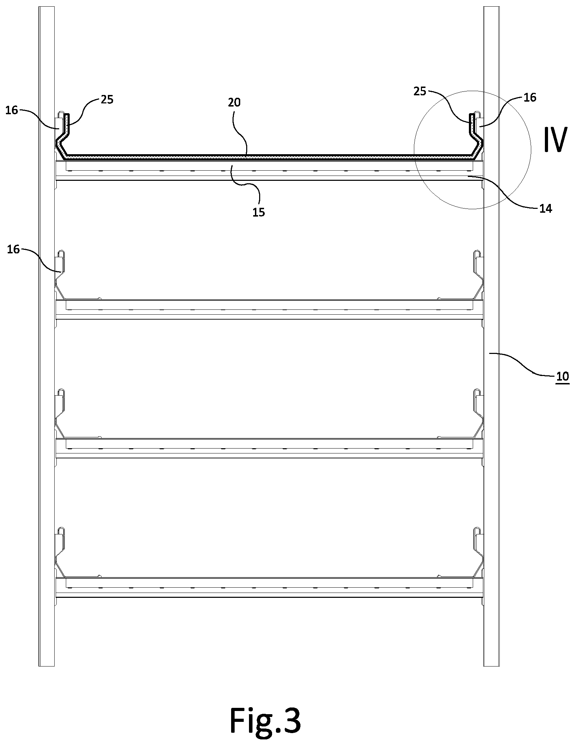

According to the invention, a plastic lining 20 is received on rack bottoms 15, see figures 2 and 3. In this embodiment use is made for this purpose of a continuous plastic cloth in the form of a flexible polyolefin film 20, for instance a polypropylene, polyethylene, ethylene propylene diene monomer (EPDM) or polyvinyl chloride (PVC). The film is introduced axially using an energizable infeed device 30 which is provided or can be provided for this purpose at the inlet/outlet 18 of rack bottoms 15, which is shown schematically in figure 2. The film 20 is for instance supplied from a roll motor-driven by infeed device 30 and propelled over the rack bottom in a flat state. A dispensing device, which scatters fresh insect larvae, optionally together with a nutrient substrate, over film 20 across a film width while film 20 is being introduced, can here be provided in or at the infeed device. This is repeated at each of the levels 11-14. Finally, a film 20 will thus lie across substantially the whole length on the relevant rack bottom 15 at each of the levels, as shown in the cross-section of figure 3.

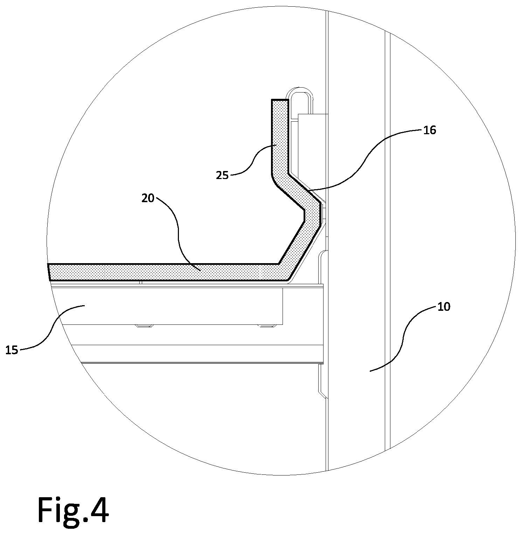

A narrowing at an outlet of infeed device 30 urges the edges of film 20 into an upward directed state at both flanks, so that the relevant edge parts 25 will be received against the upright wings 16 of rack bottom 15, see figure 3 and the enlargement of figure 4. These upward directed wing parts 16 are thus covered over at least a part of their height with the collar 25 thus formed by film 20. This collar 25 has been found to form a sufficient barrier to the insect larvae, even in an advanced stage of growth, whereby the larvae are unable to escape from the rack bottom. Tests have shown that, if they do make an attempt, the larvae cannot find purchase on the polyolefin film.

As soon as the insect larvae have developed sufficiently, the insect larvae are harvested for further processing. For this purpose a mobile outfeed device 40 is moved to the inlet/outlet 18, as shown schematically in figure 2. The outfeed device 40 pulls the film 20 off the rack bottom 15 and carries the film 20 in a flat state onto a table provided for this purpose. For this purpose the outfeed device has a motor-driven spindle (not further shown) onto which the film is then wound, while the insect larvae are scraped therefrom and are caught and collected by a collecting device (not further shown either). After being thoroughly cleaned, the cloth can then be reused by the infeed device 30.

In this embodiment the device is utilized for rearing the insect larvae of the black soldier fly. They are reared from a very early stage to a high-protein food source in a cycle of 12-15 days. A relatively small number of the reared larvae is grown further into a flying stage as soldier fly in order to produce enough eggs and new larvae for a subsequent production cycle in a hatchery. The majority is processed into a valuable protein-containing raw material which is suitable for use as a nutrient for animal and human consumption.

A particularly efficient and practical rearing system is thus provided, whereby insect larvae can be farmed in particularly effective manner and with only a small amount of manpower. Although the invention has been further elucidated above with reference to only a single exemplary embodiment, it will be apparent that the invention is by no means limited thereto. On the contrary, many variations and embodiments are still possible within the scope of the invention as set out in the appended claims, for a person with ordinary skill in the art.

Use is thus made in the example of a mobile infeed device and outfeed device which must be moved to the different levels 11-14 at a combined inlet/outlet 18. Both devices can thereby be utilized for different rack arrangements 10. Use can instead also be made of a fixed arrangement, wherein the racks have at a first end surface an inlet and at an opposite outer end an outlet, each with respectively an infeed device or outfeed and collecting device disposed optionally fixedly there.

Instead of a flexible film cloth, use can also be made for the lining of a form-retaining gutter of a plastic, particularly a polyolefin polymer. The upright wings thereof provide a similar barrier against the larvae escaping.

## Claims

1. Device for rearing insects, particularly insect larvae, more particularly fly larvae, still more particularly larvae of the black soldier fly, comprising a rack (10) with at least one elongate rack bottom (15) which extends in the rack (10) between opposite end surfaces thereof and is provided over at least substantially a whole length with a set of upward directed wings (16) on either side, **characterized in that** the rack bottom (15) is covered with a plastic lining (20), particularly a polyolefin lining (20), which also extends laterally over an upward directed part (16) of each of the wings (16), that the lining (20) is movable over the rack bottom (15) and is removable from the rack (10) at at least one of the end surfaces.

2. Device according to claim 1, **characterized in that** the rack (10) comprises a number of similar rack bottoms (15) at successive levels one above the other.

3. Device according to claim 1 or 2, **characterized in that** the rack bottoms (15) are substantially formed by a number of substantially parallel bars which extend in axial direction and maintain a mutual intermediate space.

4. Device according to one or more of the preceding claims, **characterized in that** the lining (20) comprises a continuous plastic film, particularly a polyolefin film.

5. Device according to one or more of the preceding claims, **characterized in that** the plastic is chosen from a group comprising polypropylene, polyethylene, ethylene propylene diene monomer (EPDM) and polyvinyl chloride (PVC).

6. Device according to one or more of the preceding claims, **characterized in that** the wings (16) each comprise a continuous plate body, which plate body comprises an upright wall and a lying bottom, wherein the bottom of each of the wings (16) is connected fixedly to the rack bottom (15).

7. Device according to one or more of the preceding claims, **characterized in that** at least the rack bottom (15) is formed at least substantially from a metal from a group comprising aluminium, stainless steel and optionally preserved steel.

8. Device according to one or more of the preceding claims, **characterized in that** an infeed device (30) is coupled operatively to an inlet of the rack (10), which infeed device (30) is intended and configured to carry the lining (20) axially into the rack (10) over a rack bottom (15).

9. Device according to claim 8, **characterized in that** the infeed device (30) comprises a dispensing device for supplying at least one of a food source and insect larvae onto an exposed surface of the lining (20).

10. Device according to one or more of the preceding claims, **characterized in that** an outfeed device (40) is coupled operatively to an outlet of the rack (10), which outfeed device (40) is intended and configured to carry the lining (20) axially out of the rack (10).

11. Device according to claim 10, **characterized in that** the outfeed device (40) comprises a collecting device for removing the insect larvae from the lining (20).

12. Method for rearing insect larvae in a rearing device, wherein at least one device according to one or more of the preceding claims is utilized as rearing device.

## Patentansprüche

1. Vorrichtung zur Aufzucht von Insekten, insbesondere Insektenlarven, insbesondere Fliegenlarven, ganz insbesondere Larven der Schwarzen Soldatenfliege, umfassend ein Gestell (10) mit mindestens einem länglichen Gestellboden (15), der sich im Gestell (10) zwischen dessen gegenüberliegenden Endflächen erstreckt und über mindestens im Wesentlichen einer gesamten Länge mit einem Satz nach oben gerichteter Flügel (16) auf jeder Seite versehen ist, **dadurch gekennzeichnet, dass** der Gestellboden (15) mit einer Kunststoffauskleidung (20), insbesondere einer Polyolefinauskleidung (20), bedeckt ist, die sich auch seitlich über einen nach oben gerichteten Teil (16) jedes der Flügel (16) erstreckt, dass die Auskleidung (20) über den Gestellboden (15) bewegbar und an mindestens einer der Stirnflächen vom Gestell (10) abnehmbar ist.

2. Vorrichtung nach Anspruch 1, **dadurch gekennzeichnet, dass** das Gestell (10) eine Anzahl ähnlicher Gestellböden (15) aufeinanderfolgend übereinander angeordnet umfasst.

3. Vorrichtung nach Anspruch 1 oder 2, **dadurch gekennzeichnet, dass** die Gestellböden (15) im Wesentlichen aus einer Anzahl von im Wesentlichen parallelen Stangen gebildet sind, die sich in axialer Richtung erstrecken und einen gegenseitigen Zwischenraum einhalten.

4. Vorrichtung nach einem oder mehreren der vorstehenden Ansprüche, **dadurch gekennzeichnet, dass** die Auskleidung (20) eine durchgehende Kunststofffolie, insbesondere eine Polyolefinfolie, umfasst.

5. Vorrichtung nach einem oder mehreren der vorstehenden Ansprüche, **dadurch gekennzeichnet, dass** der Kunststoff aus einer Gruppe ausgewählt ist, die Polypropylen, Polyethylen, Ethylen-Propylen-Dien-Monomer (EPDM) und Polyvinylchlorid (PVC) umfasst.

6. Vorrichtung nach einem oder mehreren der vorstehenden Ansprüche, **dadurch gekennzeichnet, dass** die Flügel (16) jeweils einen durchgehenden Plattenkörper umfassen, wobei der Plattenkörper eine aufrecht stehende Wand und einen liegenden Boden umfasst, wobei der Boden jedes der Flügel (16) fest mit dem Gestellboden (15) verbunden ist.

7. Vorrichtung nach einem oder mehreren der vorstehenden Ansprüche, **dadurch gekennzeichnet, dass** zumindest der Gestellboden (15) zumindest im Wesentlichen aus einem Metall aus einer Gruppe bestehend aus Aluminium, Edelstahl und gegebenenfalls konserviertem Stahl gebildet ist.

8. Vorrichtung nach einem oder mehreren der vorstehenden Ansprüche, **dadurch gekennzeichnet, dass** eine Zuführvorrichtung (30) funktionsfähig mit einem Einlass des Gestells (10) verbunden ist, wobei die Zuführvorrichtung (30) dazu bestimmt und konfiguriert ist, die Auskleidung (20) axial über einen Gestellboden (15) in das Gestell (10) zu befördern.

9. Vorrichtung nach Anspruch 8, **dadurch gekennzeichnet, dass** die Zuführvorrichtung (30) eine Abgabevorrichtung zum Zuführen mindestens einer Nahrungsquelle und/oder Insektenlarven auf eine freiliegende Oberfläche der Auskleidung (20) umfasst.

10. Vorrichtung nach einem oder mehreren der vorstehenden Ansprüche, **dadurch gekennzeichnet, dass** eine Ausgabevorrichtung (40) operativ mit einem Auslass des Gestells (10) verbunden ist, wobei die Ausgabevorrichtung (40) dazu bestimmt und konfiguriert ist, die Auskleidung (20) axial aus dem Gestell (10) herauszutransportieren.

11. Vorrichtung nach Anspruch 10, **dadurch gekennzeichnet, dass** die Ausgabevorrichtung (40) eine Sammelvorrichtung zum Entfernen der Insektenlarven von der Auskleidung (20) umfasst.

12. Verfahren zur Aufzucht von Insektenlarven in einer Aufzuchtvorrichtung, wobei mindestens eine Vorrichtung gemäß einem oder mehreren der vorstehenden Ansprüche als Aufzuchtvorrichtung verwendet wird.

## Revendications

1. Dispositif d'élevage d'insectes, en particulier de larves d'insectes, plus particulièrement de larves de mouches, encore plus particulièrement de larves de la mouche soldat noire, comprenant un panier (10) avec au moins un fond de panier allongé (15) qui s'étend dans le panier (10) entre ses surfaces d'extrémité opposées et qui est pourvu sur au moins la quasi-totalité de sa longueur d'un ensemble d'ailes (16) orientées vers le haut de chaque côté, **caractérisé par le fait que** le fond de panier (15) est recouvert d'un revêtement en plastique (20), en particulier un revêtement en polyoléfine (20), qui s'étend également latéralement sur une partie orientée vers le haut (16) de chacune des ailes (16), que le revêtement (20) est mobile sur le fond du casier (15) et qu'il est amovible du casier (10) au niveau d'au moins une des surfaces d'extrémité.

2. Dispositif selon la revendication 1, **caractérisé par le fait que** la panier (10) comprend plusieurs fonds de panier (15) similaires à des niveaux successifs l'un au-dessus de l'autre.

3. Dispositif selon la revendication 1 ou 2, **caractérisé par le fait que** les fonds de panier (15) sont essentiellement formés par un certain nombre de barres sensiblement parallèles qui s'étendent dans la direction axiale et maintiennent un espace intermédiaire mutuel.

4. Dispositif selon l'une ou plusieurs des revendications précédentes, **caractérisé par le fait que** le revêtement (20) est constitué d'un film plastique continu, notamment d'un film de polyoléfine.

5. Dispositif selon une ou plusieurs des revendications précédentes, **caractérisé par le fait que** la matière plastique est choisie dans un groupe comprenant le polypropylène, le polyéthylène, l'éthylène propylène diène monomère (EPDM) et le polychlorure de vinyle (PVC).

6. Dispositif selon l'une ou plusieurs des revendications précédentes, **caractérisé par le fait que** les ailes (16) comprennent chacune un corps de plaque continu, le corps de plaque comprenant une paroi verticale et un fond couché, le fond de chacune des ailes (16) étant relié de manière fixe au fond de la panier (15).

7. Dispositif selon l'une ou plusieurs des revendications précédentes, **caractérisé par le fait qu'**au moins le fond du panier (15) est formé au moins en grande partie d'un métal appartenant à un groupe comprenant l'aluminium, l'acier inoxydable et éventuellement l'acier préservé.

8. Dispositif selon l'une ou plusieurs des revendications précédentes, **caractérisé par le fait qu'**un dispositif d'alimentation (30) est couplé de manière opérationnelle à une entrée du panier (10), ce dispositif d'alimentation (30) étant destiné et configuré pour transporter le revêtement (20) axialement dans le panier (10) au-dessus d'un fond de panier (15).

9. Dispositif selon la revendication 8, **caractérisé par le fait que** le dispositif d'alimentation (30) comprend un dispositif de distribution pour fournir au moins une source de nourriture et des larves d'insectes sur une surface exposée du revêtement (20).

10. Dispositif selon l'une ou plusieurs des revendications précédentes, **caractérisé par le fait qu'**un dispositif de sortie (40) est couplé de manière opérationnelle à une sortie du panier (10), ce dispositif de sortie (40) étant destiné et configuré pour transporter le revêtement (20) axialement hors du panier (10).

11. Dispositif selon la revendication 10, **caractérisé par le fait que** le dispositif de sortie (40) comprend un dispositif de collecte pour retirer les larves d'insectes du revêtement (20).

12. Procédé d'élevage de larves d'insectes dans un dispositif d'élevage, au moins un dispositif selon l'une ou plusieurs des revendications précédentes étant utilisé comme dispositif d'élevage.
